(19) Europäisches Patentamt
European Patent Office
Office européen des brevets

(11) Veröffentlichungsnummer: **0 166 122**
**B1**

(12) # EUROPÄISCHE PATENTSCHRIFT

(45) Veröffentlichungstag der Patentschrift: **12.04.89**

(51) Int. Cl.⁴: **A 61 K 7/06**, A 61 K 7/11, C 08 G 77/38

(21) Anmeldenummer: **85105323.1**

(22) Anmeldetag: **02.05.85**

(54) **Betaingruppen enthaltende Siloxane, deren Herstellung und Verwendung in kosmetischen Zubereitungen.**

(30) Priorität: **15.05.84 DE 3417912**

(43) Veröffentlichungstag der Anmeldung: **02.01.86 Patentblatt 86/1**

(45) Bekanntmachung des Hinweises auf die Patenterteilung: **12.04.89 Patentblatt 89/15**

(84) Benannte Vertragsstaaten: **BE DE FR GB IT NL**

(56) Entgegenhaltungen:
**EP-A- 0 017 121**
**GB-A- 2 113 245**

**Die Akte enthält technische Angaben, die nach dem Eingang der Anmeldung eingereicht wurden und die nicht in dieser Patentschrift enthalten sind.**

(73) Patentinhaber: **Th. Goldschmidt AG, Goldschmidtstrasse 100 Postfach 101461, D-4300 Essen 1 (DE)**

(72) Erfinder: **Hoffmann, Klaus, Ahornstrasse 47, D-4300 Essen (DE)**
Erfinder: **Kollmeier, Hans-Joachim, Dr., Barkhorstrücken 27, D-4300 Essen (DE)**
Erfinder: **Langenhagen, Rolf-Dieter, Kampstrasse 5, D-4321 Hattingen-Niederwenigern (DE)**

EP 0 166 122 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neuartige Organopolysiloxane mit Betaingruppen und Verfahren zur Herstellung dieser Verbindungen. Sie betrifft schliesslich die Verwendung dieser Verbindungen in kosmetischen Zubereitungen.

Es ist bekannt, Organopolysiloxane zur Herstellung von Haarpflegemitteln zu verwenden. In «Chemie und Technologie der Silicone» von Walter Noll, Verlag Chemie, 2. Auflage, 1968, Seite 536, heisst es allerdings, dass die Aufgabe, die Frisur unabhänig von Feuchtigkeitseinflüssen zu erhalten, mit normalen Polydimethylsiloxanolen nicht zu lösen sei. Das Silicon müsse vielmehr mit Hilfe von funktionellen Gruppierungen auf dem Haar fixiert werden.

Aus der DE-AS 1 493 384 sind Organosiloxanverbindungen oder -verbindungsgemische der Formel

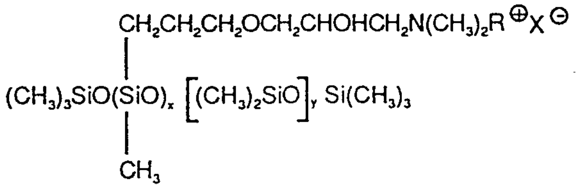

in der R für Wasserstoff oder $CH_3$ und X für Halogen steht und x = 1 bis 10 und y = 0 bis 8,5 sind, wobei y : x nicht grösser als 8,5 : 1 ist, bekannt.

Diese Organosiloxane mit quaternären Ammoniumgruppen können dadurch hergestellt werden, dass man eine Epoxysiloxanverbindung der Formel

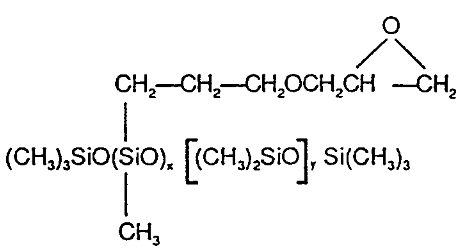

auf an sich bekannte Weise mit Dimethylamin umsetzt und die erhaltene Dimethylaminoorganosiloxanverbindung der Formel

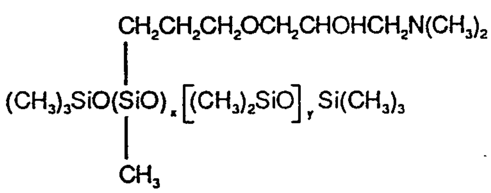

in an sich bekannter Weise mit einem Halogenwasserstoff oder mit einem Methylhalogenid in die quaternäre Ammoniumverbindung der vorgenannten Formel überführt.

Die vorgenannten Organopolysiloxane mit quaternären Ammoniumgruppen können entsprechend der US-PS 4 185 087 für Haarpflegemittel verwendet werden. Dort ist ausgeführt, dass zwar einfache wässrige Haarwaschmittel das Haar von Schmutz befreien und einen Überschuss an Fett entfernen könnten. Bei den meisten Haarwaschmitteln würde jedoch die Entfettung des Haares so gründlich vorgenommen, dass eine Schädigung des Haares zu beobachten sei. Die Haare würden sich nach der Wäsche elektrostatisch aufladen und deshalb schlecht kämmbar sein. Der Zusatz von Lanolinderivaten, Glykol, Fettsäureestern oder Proteinen verbessere zwar die Handhabbarkeit des Haares nach dem Waschen, beeinträchtigte aber gleichzeitig die Schaumbildung. Die Haare würden etwa klebrig und fühlten sich unnatürlich an. Die vorgeschriebenen Organopolysiloxane mit quaternären Ammoniumgruppen sollen nach den Angaben der US-PS 4 185 087 diese Nachteile beseitigen und die Kämmbarkeit der gewaschenen Haare, den Halt der Frisur, den Glanz der Haare verbessern.

Eine ähnliche Lehre ergibt sich aus den europäischen Patentschriften 0 017 121 und 0 017 122. Auch hier sind Organopolysiloxane mit quaternären Ammoniumgruppen in Haarwasch- und Haarbehandlungsmitteln zur Verbesserung der frisiertechnischen Eigenschaften der Haare beschrieben. Die Verbindungen entsprechen dabei der allgemeinen Formel

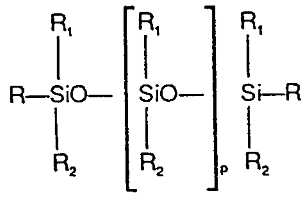

in der $R_1$ und $R_2$ einen Alkylrest mit 1 bis 4 Kohlenstoffatomen oder einen Arylrest, p die Zahlen 0 bis 50 und R die Reste

a) 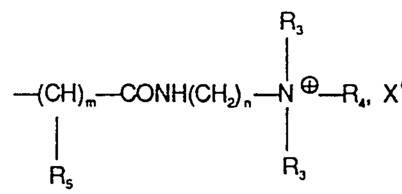 oder

b) 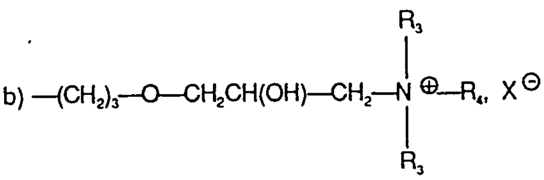

darstellen, wobei $R_3$ einen Alkyl- oder Hydroxy-alkylrest mit 1 bis 3 Kohlenstoffatomen, $R_4$ einen Rest gleich $R_3$ oder Aryl-$CH_2$- oder den Allylrest, $R_5$ Wasserstoff oder den Methylrest, $X^\ominus$ die Anionen $Cl^\ominus$, $Br^\ominus$, $J^\ominus$, $CH_3SO_4^\ominus$ oder $C_2H_5SO_4^\ominus$ und m die Zahlen 2 bis 10, n die Zahlen 2 bis 4 bedeuten.

Schliesslich sei noch auf die veröffentlichte europäische Patentanmeldung 0 095 238 verwiesen, welche eine Zusammensetzung betrifft, die im wesentlichen aus folgenden Bestandteilen besteht:

A) einem Siloxan der allgemeinen Formel

$$R_aX_{3-a}Si(OSiX_2)_n(OSiX_bR_{2-b})_mOSiX_{3-a}R_a$$

wobei R nur aufgabenhaft als eine funktionelle Gruppe angegeben ist, welche die Haftung am Haar bewirkt, z.B. eine Amino-, Carbonsäure-oder quaternäre Ammoniumgruppe. X ist ein Wasserstoffrest oder eine Phenyl-, Hydroxyl- oder gesättigte Kohlenwasserstoffgruppe mit 1 bis 8 Kohlenstoffatomen. a hat einen Wert von 0 bis 3, b hat einen Wert von 0 bis 1 und n + m hat einen Wert von 1 bis 1999, wobei n einen Wert von 0 bis 2000 und m einen Wert von 1 bis 2000 hat.

B) einem Tensid,

C) einem Zusatz zur Verbesserung der Gefrier/Auftau-Stabilität und

D) Wasser.

Aus dem Stand der Technik ergibt sich somit, dass die Organopolysiloxane mit quaternären Ammoniumgruppen eine starke Substantivität auf dem Haar aufweisen und ihm gute Kämmbarkeit und Glanz verleihen. Ein Nachteil ist jedoch deren schlechte Verträglichkeit mit anionischen Komponenten, insbesondere mit anionischen Tensiden, in Haarpflegezubereitungen. Sie können darüber hinaus auch zu Irritationen der Haut, insbesondere der Schleimhaut und im Auge, führen. Dies ist aber gerade bei Haarwaschmitteln äusserst unerwünscht.

Der Erfindung liegt die Aufgabe zugrunde, Wirkstoffe für Haarkosmetika zu finden, welche Substantivität gegenüber dem Haar aufweisen, dem behandelten Haar gute Kämmbarkeit und guten Glanz verleihen, keine Hautirritationen verursachen und darüber hinaus mit anionischen Detergentien verträglich sind.

Überraschenderweise wurde gefunden, dass diese Eigenschaften bei Organopolysiloxanen zu finden sind, welche eine oder mehrere Betaingruppen aufweisen. Gegenstand der Erfindung sind somit zunächst Verbindungen der allgemeinen Formel

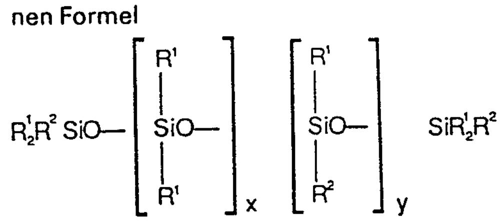

wobei

$R^1$ im Molekül gleich oder verschieden ist und einen Alkylrest mit 1 bis 18 Kohlenstoffatomen, einen Arylrest oder einen Polyoxyalkylenrest der allgemeinen Formel $-(CH_2)_3O(C_mH_{2m}O)_pQ$ bedeutet, wobei die Gruppe $-(C_mH_{2m}O)_p$ aus Oxyethylen- und Oxypropyleneinheiten aufgebaut ist, m einen durchschnittlichen Wert von 2,0 bis 2,6, p einen Wert von 1 bis 25 hat, Q ein Wasserstoff- oder ein Alkylrest mit 1 bis 4 Kohlenstoffatomen ist, jedoch mindestens 70% der Reste $R^1$ Methylreste sind,

$R^2$ gleich $R^1$ sein kann, wobei mindestens ein Rest $R^2$ die Gruppe

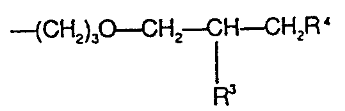

ist, in der $R^3$ und $R^4$ verschieden sind und ein Rest ein Hydroxylrest und der andere Rest die Gruppe

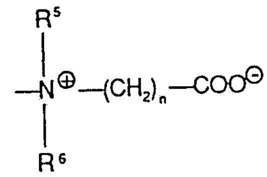

ist, in der $R^5$ und $R^6$ gleich oder verschieden sind und einen Alkylrest mit 1 bis 4 Kohlenstoffatomen oder einen Benzylrest bedeuten, und n = 1, 2 oder 3 ist,

x einen Wert von 0 bis 200 und

y einen Wert von 1 bis 50 hat.

Aus der allgemeinen Formel I ist ersichtlich, dass die Betaingruppe(n) endständig oder seitenständig gebunden sein kann (können).

Mindestens 70% der Reste $R^1$ sind Methylreste. Besonders bevorzugt sind solche Polysiloxane, bei denen alle Reste $R^1$ Methylreste sind. Es können jedoch bis zu 30% der Reste $R^1$ Alkylreste mit 2 oder mehr, vorzugsweise aber 12 bis 18 Kohlenstoffatomen oder Arylreste sein. Beispiele solcher Alkylreste sind der Ethyl-, Propyl-, Isopropyl-, Butyl-, Hexyl-, Isooctyl-, Decyl-, Dodecyl- oder Stearylrest. Der Arylrest ist im allgemeinen ein Phenylrest. Sowohl die Alkylreste wie die Arylreste können substituiert sein. Bevorzugt sind Verbindungen, in denen 3 bis 10% der Reste $R^1$ Polyoxyalkylenreste sind.

Mindestens ein Rest $R^2$ muss die Bedeutung der Gruppe

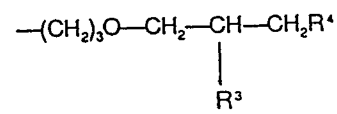

haben. Dabei ist einer der Reste $R^3$ und $R^4$ ein Hydroxylrest. Der andere Rest ist die Gruppe

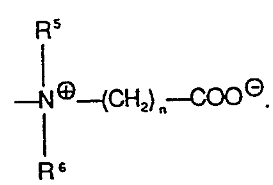

. $R^5$ und $R^6$ sind dabei vorzugsweise Methylreste. Sie können jedoch auch Ethyl-, Propyl-, Isopropyl- oder Butylreste sein oder einen Benzylrest bedeuten. n hat einen Wert von 1, 2 oder 3, jedoch ist n = 1 bevorzugt.

x hat vorzugsweise einen Wert von 2 bis 100, insbesondere einen Wert von 5 bis 50.

y hat einen Wert von 1 bis 25, insbesondere einen Wert von 2 bis 10.

Beispiele erfindungsgemässer Organopolysiloxane mit Betaingruppen sind

a)

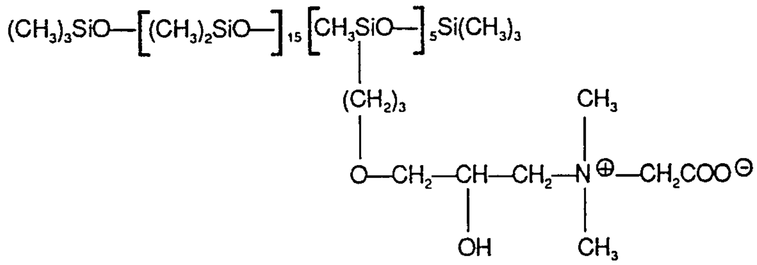

b)

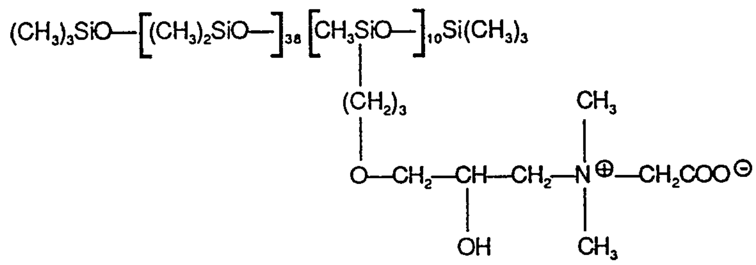

c)

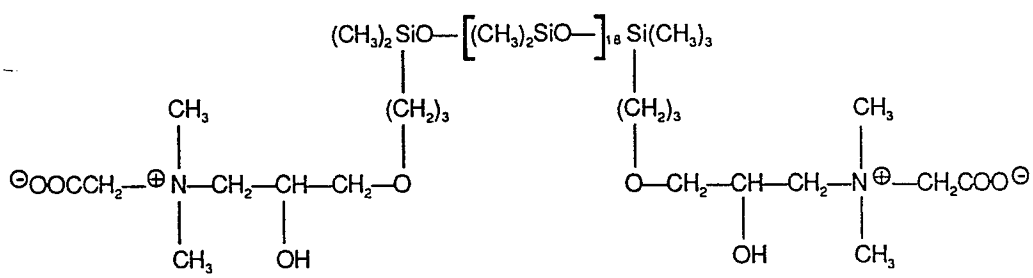

d)

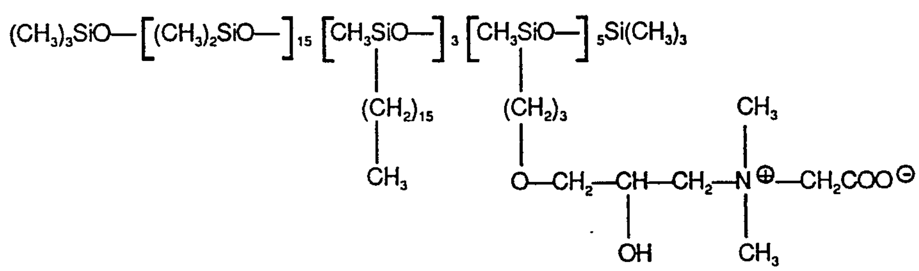

e)

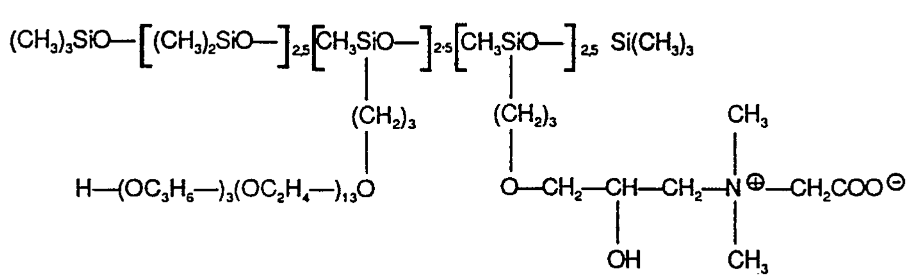

Die erfindungsgemässen Verbindungen sind im allgemeinen viskose bis hochviskose, ölige bis pastenartige und farblose bis gelb gefärbte Produkte. Die Löslichkeit der erfindungsgemässen Verbindungen wird im wesentlichen durch das Verhältnis der Anzahl der Betaingruppen zu der Anzahl der Siloxyeinheiten ($R^1_2SiO$) sowie der Art der Reste $R^1$ bestimmt. Höhere Gehalte an Betaingruppen und/oder die Anwesenheit von Polyoxyalkylenresten mit überwiegendem Anteil von Oxyethyleneinheiten ergeben Produkte, die in Wasser und niedrigen Alkoholen oder Glykolen löslich sind. Durch den Einbau von Alkylresten mit 12 bis 18 Kohlenstoffatomen können demgegenüber ölverträgliche bzw. öldispergierbare Produkte erhalten werden. Für die Anwendung in Haarpflegemitteln sind im allgemeinen Produkte bevorzugt, die in Wasser oder Glykolen löslich sind.

Ein weiterer Gegenstand der Erfindung ist die Herstellung der erfindungsgemässen Verbindungen. Die Verfahren zur Herstellung der erfindungsgemässen Organopolysiloxane mit Betaingruppen bestehen darin, dass man entweder

a) Verbindungen der allgemeinen Formel

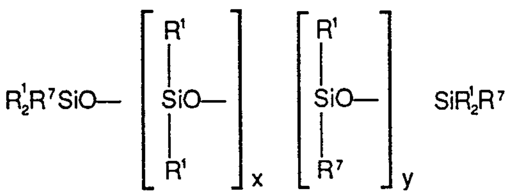
II

wobei $R^7$ gleich $R^1$ sein kann, mit der Massgabe, dass mindestens ein Rest $R^7$ die Gruppe

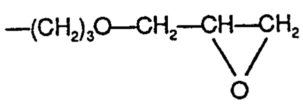

ist, mit, bezogen auf die Gruppe

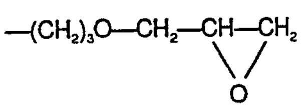

äquimolaren Mengen Verbindungen der allgemeinen Formel

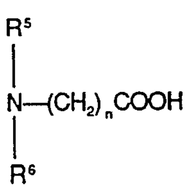

in an sich bekannter Weise umsetzt, oder

b) Verbindungen der allgemeinen Formel

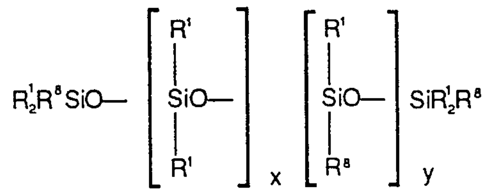
III

wobei $R^8$ gleich $R^1$ sein kann, mit der Massgabe, dass mindestens ein Rest $R^8$ die Gruppe

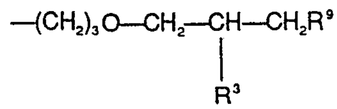

ist, in der $R^3$ und $R^9$ verschieden sind, und ein Rest ein Hydroxylrest und der andere Rest die Gruppe

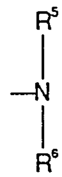

ist, mit, bezogen auf die Gruppe

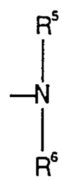

äquimolaren Mengen Verbindungen der allgemeinen Formel $X-(CH_2)_n-COOY$, wobei X ein Chlor- oder Bromrest und Y ein Alkalirest ist, in an sich bekannter Weise umsetzt.

Die Herstellung der Verbindungen der Formel II und III, welche beim erfindungsgemässen Verfahren als Ausgangsverbindungen dienen, ist dem Fachmann aus dem Stand der Technik geläufig und nicht Gegenstand vorliegender Erfindung. Man kann beispielsweise an Wasserstoffsiloxane, die der Formel III entsprechen, wobei jedoch die Reste $R^7$ Wasserstoffreste sind, die entsprechenden Allylverbindungen, z. B.

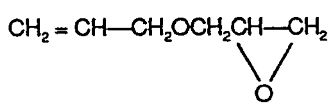

addieren und zur Herstellung der Verbindungen III diese zunächst erhaltenen Additionsprodukte mit Aminen der Formel $HNR^5R^6$ umsetzen.

Die Umsetzung der Verbindungen der allgemeinen Formel II mit Verbindungen der allgemeinen Formel

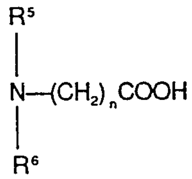

bzw. die Umsetzung der Verbindungen der allgemeinen Formel III mit den Verbindungen der allgemeinen Formel X-$(CH_2)_n$-COOY erfolgt in jeweils an sich bekannter Weise. Vorzugsweise wird die Umsetzung bei erhöhten Temperaturen, insbesondere in einem Temperaturbereich von 40 bis 160°C, durchgeführt. Man kann die Umsetzung auch in Gegenwart von Lösungsmitteln vornehmen, wobei die obere Temperaturgrenze dann durch den Siedepunkt des verwendeten Lösungsmittels gegeben ist. Beispiele geeigneter Lösungsmittel sind Wasser, niedrige Alkohole oder Glykole.

Die erfindungsgemässen Verbindungen zeigen die gewünschte Eigenschaftskombination. Der Vergleich mit nach dem Stand der Technik verwendeten ähnlichen Produkten kann der folgenden Tabelle entnommen werden.

Tabelle

| Wirkstoff | Substantivität auf Haar | Kämmbarkeit und Glanz der Haare | Hautirritation | Verträglichkeit mit anionischen Wirkstoffen |
|---|---|---|---|---|
| Siloxan mit Polyethergruppen | schwach | schwach | keine | gut |
| Siloxan mit anionischen Gruppen | keine | keine | schwach | gut |
| Siloxan mit Aminogruppen | mittel | mittel | mittel | gut |
| Siloxan mit quaternären Aminogruppen | stark | gut | schwach | schlecht |
| Siloxan mit Betaingruppen | mittel | gut | keine | gut |
| siloxanfreies Betain | mittel | schwach | keine | gut |

Aus der Tabelle ist ersichtlich, dass die Organopolysiloxane mit Betaingruppen die gewünschte Eigenschaftskombination aufweisen, die keine der übrigen Verbindungen zeigt.

Es ist deshalb ein weiterer Gegenstand der Erfindung, die erfindungsgemässen Verbindungen in kosmetischen Zubereitungen, insbesondere in Zubereitungen zur Pflege der Haare, zu verwenden. Haarkosmetika können dabei Haarwaschmittel oder Haarpflegemittel sein, je nachdem ob der Reinigungseffekt oder der Pflegeeffekt und der Effekt der besseren Kämmbarkeit im Vordergrund steht. Haarwaschmittel, denen die erfindungsgemässen Organopolysiloxane mit Betaingruppen in Mengen von 0,1 bis 10 Gew.-% zugesetzt sind und die bis zu 30 Gew.-% waschaktiver Substanzen neben Wasser und gegebenenfalls anderen Zusatzstoffen enthalten, bewirkten, dass die gewaschenen Haare Fülle und angenehmen Griff aufweisen, den gewünschten Glanz zeigen und leicht kämmbar sind. Eine elektrostatische Aufladung der Haare ist praktisch nicht zu beobachten. In Haarpflegemitteln, wie Haarwässern oder Haarsprays, bewirken die erfindungsgemässen Verbindungen in Mengen von 0,1 bis 5% bereits eine wesentliche Verbesserung der Kämmbarkeit der Haare und bilden Fülle und Glanz aus.

Den Haarpflegemitteln können übliche Zusatzmittel, wie Lösungsmittel, Verdickungsmittel, Parfüm, Konservierungsmittel, Komplexbildner, Schaumstabilisierungsmittel, Trübungsmittel, Perlglanzmittel oder andere übliche Zusatzstoffe, wie Farbstoffe, zugegeben werden. Rezepturbeispiele sind:

| | |
|---|---|
| Mittel für eine Cremekurspülung | |
| Cetylalkohol | 6 Gew.-Teile |
| Teginacid®H* | 6 Gew.-Teile |
| Glycerin | 3 Gew.-Teile |
| Betain-Siloxan | |
| (gemäss Beispiel 1) | 1 Gew.-Teil |
| Wasser | 84 Gew.-Teile |
| *Handelsprodukt der Th. Goldschmidt AG | |

| | |
|---|---|
| Konditioniershampoo | |
| a) Natriumlaurylethersulfat | 3 Gew.-Teile |
| Ammoniumalkylethersulfat | 6 Gew.-Teile |
| Tagat® KL 141* | 5 Gew.-Teile |
| Betain-Siloxan | |
| (gemäss Beispiel 1) | 2 Gew.-Teile |
| Wasser | 84 Gew.-Teile |
| b) Kokosfettsäurediethanolamid | 0,5 Gew.-Teile |
| Natriumlaurylethersulfat | 30 Gew.-Teile |
| Kochsalz | 1,5 Gew.-Teile |
| Tego®-Betain L 7* | |
| (Alkylamidobetain) | 8 Gew.-Teile |
| Betain-Siloxan | |
| (gemäss Beispiel 1) | 2 Gew.-Teile |
| Wasser | 58 Gew.-Teile |

Die erfindungsgemässen Verbindungen können auch Hautpflegemitteln zugegeben werden. Als Bestandteil von Seifen oder Hautcremes bilden sie auf der Haut einen feinen, nicht hautreizenden, nicht fettenden Film aus. Im Gegensatz zu niedrigviskosen, insbesondere cyclischen Dimethylsiloxanen verdunsten sie nicht auf der Haut und ergeben daher einen beständigen Schutz.

Dabei kann eine flüssige Seife folgendermassen zusammengesetzt sein:

| | |
|---|---|
| Tagat® O 2* | 1 Gew.-Teil |
| Kokosfettsäurediethanolamid | 0,5 Gew.-Teile |
| Natriumlaurylethersulfat | 30 Gew.-Teile |
| Tego®-Betain L 7* | |
| (Alkylamidobetain) | 7 Gew.-Teile |
| Kochsalz | 2 Gew.-Teile |
| Betain-Siloxan | |
| (gemäss Beispiel 2) | 2 Gew.-Teile |
| Wasser | 57,5 Gew.-Teile |
| *Handelsprodukt der Th. Goldschmidt AG | |

In den folgenden Beispielen wird das erfindungsgemässe Verfahren näher erläutert. Es werden ferner anwendungstechnische Überprüfungen im Vergleich mit Produkten des Standes der Technik gezeigt.

Beispiel 1

In einem Autoklaven werden 214,6 g (= 0,1 Mol) eines Siloxans der durchschnittlichen Zusammensetzung

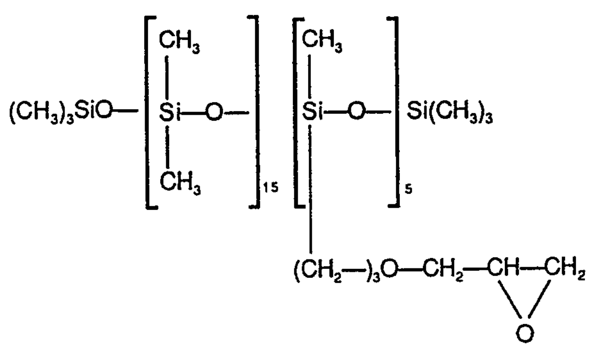

und 110 g (ca. 100% Überschuss) einer 40%igen, wässrigen Dimethylaminlösung unter Rühren 3 Std. bei 120 °C umgesetzt. Der Druck steigt dabei auf ca. 4,5 bar an. Das erhaltene gelbgefärbte, zweiphasige Reaktionsgemisch wird durch Destillation von Wasser und überschüssigem Amin befreit (Badtemperatur: bis 90 °C, Druck: 20 mbar). Der erhaltene bräunlich gefärbte, flüssige Rückstand ist schwach trübe und wird filtriert. Man erhält ein klares Produkt mit einem N-Gehalt von 2,85% (theoretisch: 2,95%).

In einem mit Thermometer, Rührer und Rückflusskühler versehenen Dreihalskolben werden 172 g (= 0,35 Mol Aminogruppen) des zuvor hergestellten, teritäre Aminogruppen enthaltenden Siloxans mit 40,8 g (= 0,35 Mol) $ClCH_3COONa$ in 230 g Wasser 5 Std. bei 100 °C gerührt. Nach ca. 1 Std. wird der anfangs trübe Ansatz klar. Nach der Reaktion lassen sich in der Lösung 2,75% ionogenes Chlor bestimmen, was einem Umsatz von 98,2% entspricht. Anschliessend wird das Wasser in einem Rotationsverdamper abdestilliert (Badtemperatur: 90 °C, Druck: 20 mbar). Es verbleibt ein trüber Rückstand, der bei 90 °C noch fliessfähig und bei Raumtemperatur fast fest ist. Um das entstehende Natriumchlorid zu entfernen, wird der Rückstand in 150 ml i-Propanol aufgenommen und filtriert. Anschliessend wird erneut in einem Rotationsverdampfer eingeengt. Man erhält ein klares, hellbraunes Produkt, das bei Raumtemperatur kaum fliessfähig ist. Die Analyse ergibt einen Gehalt von 2,4% Betain-Stickstoff (theoretisch: 2,55%).

Von der Substanz werden 1 und 0,1%ige Lösungen in Wasser hergestellt und die Oberflächenspannungen bei 20 °C gemessen:
1%ige Lösung: 28,1 $mN \cdot m^{-1}$
0,1%ige Lösung: 29,0 $mN \cdot m^{-1}$

Beispiel 2

In einem mit Thermometer, Rührer und Rückflusskühler versehenen Kolben werden 140 g einer 33%igen, ethanolischen Dimethylaminlösung bei 20 °C vorgelegt. In 30 Min. werden 236,2 g (= 0,05 Mol) eines Siloxans der durchschnittlichen Zusammensetzung

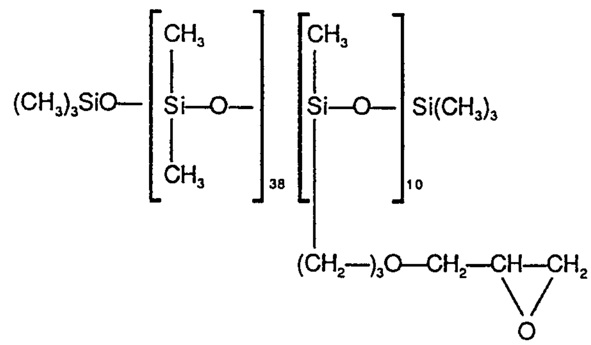

zugetropft. Dabei steigt die Temperatur auf etwa 40 °C an. Nach 1 Std. wird auf Rückflusstemperatur aufgeheizt und weitere 5 Std. gerührt. Die klare, gelbliche Lösung wird von überschüssigem Amin und Ethanol in einem Rotationsverdampfer befreit (Badtemperatur: bis 80 °C, Druck: 22 mbar). Man erhält als Rückstand eine klare, gelbe, leicht viskose Flüssigkeit. Der N-Gehalt beträgt 2,7% (theoretisch: 2,71%).

In einen mit Thermometer, Rückflusskühler und Rührer versehenen Dreihalskolben werden 207,5 g (= 0,4 Mol Aminogruppen) des erhaltenen, tertiäre Aminogruppen enthaltenden Siloxans mit 46,6 g (= 0,4 Mol) $ClCH_2COONa$ in 207,3 g Wasser und 307,6 g 1.2-Propylenglykol gegeben und 5 Std. bei 100 °C gerührt. Der Ansatz wird nach ca. 1 Std. klar. Nach der Reaktion lassen sich in der klaren, gelben, leicht viskosen Lösung 1,8% ionogenes Chlor bestimmen, was einem Umsatz von 97,8% entspricht. Ausserdem ergibt die Analyse in der Lösung einen Gehalt von 0,7% Betain-Stickstoff (theoretisch: 0,73%).

Die erhaltene Lösung besteht zu 30% aus dem gewünschten Betaingruppen enthaltenden Siloxan, zu 3% aus NaCl, zu 27% aus Wasser und zu 40% aus 1.2-Propylenglykol.

Durch Verdünnen mit Wasser werden 1 und 0,1%ige Lösungen hergestellt und ihre Oberflächenspannungen (bei 20 °C) bestimmt:
1%ige Lösung: 25,5 $mN \cdot m^{-1}$
0,1%ige Lösung: 26,5 $mN \cdot m^{-1}$

Beispiel 3

In einem mit Thermometer, Rührer und Rückflusskühler versehenen Kolben werden 70 g einer 33%igen, ethanolischen Dimethylaminlösung bei Raumtemperatur vorgelegt. In 25 Min. werden 372,2 g (= 0,1 Mol) eines Siloxans der durchschnittlichen Zusammensetzung

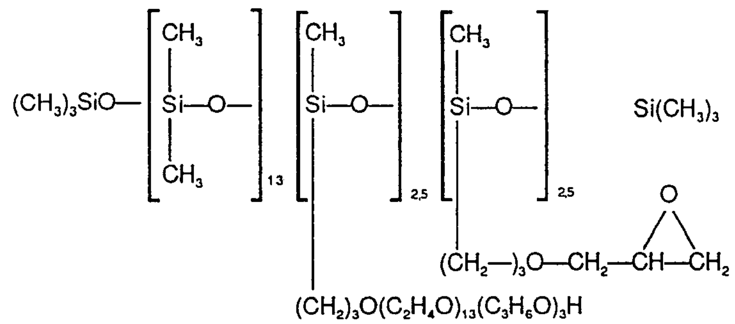

zugetropft. Dieses, neben Epoxygruppen nach Polyoxyalkylenreste aufweisende Siloxan lässt sich nach bekanntem Verfahren durch Anlagerung von $CH_2 = CH\text{-}CH_2\text{-}O(C_2H_4O\text{-})_{13}$ $(C_3H_6O\text{-})_3H$ und Allylglycidether an das entsprechende SiH-Gruppen tragende Siloxan $(CH_3)_3SiO\,[(CH_3)_2SiO]_{13}\,[(CH_3)HSiO]_5Si(CH_3)_3$ in Gegenwart von Platinkatalysatoren herstellen.

Nach der Zugabe des Siloxans zur Dimethylaminlösung wird auf 75°C erwärmt und 6 Std. gerührt. Im Anschluss werden überschüssiges Amin und Ethanol in einem Rotationsverdampfer abdestilliert (Badtemperatur: bis 80°C, Druck: 15 mbar). Man erhält ein klares, gelbgefärbtes, viskoses Produkt, das einen N-Gehalt von 0,87% hat (theoretisch: 0,91%.

In einem mit Thermometer, Rührer und Rückflusskühler versehenen Dreihalskolben werden 322 g (= 0,2 Mol Aminogruppen) des erhaltenen Produktes mit 23,3 g (= 0,2 Mol) $ClCH_2COONa$ in 321,9 g Wasser und 166,8 g 1.2-Propylenglykol gegeben und 6 Std. bei 100°C gerührt. Nach ca. 15 Min. wird der Ansatz klar. Nach der Reaktion lassen sich in der klaren, gelben Lösung 0,8% ionogenes Chlor nachweisen, was einem Umsatz von 94,1% entspricht. Ausserdem ergibt die Analyse einen Gehalt von 0,31% Betain-Stickstoff (theoretisch: 0,34%).

Die erhaltene Lösung besteht zu 40% aus einem mit Betaingruppen und Polyoxyalkylenresten modifizierten Siloxan, zu 1,4% aus NaCl, zu 38,6% aus Wasser und zu 20% aus 1.2-Propylenglykol.

Durch Verdünnen mit Wasser werden 1 und 0,1%ige Lösungen zur Messung von Oberflächenspannungen (bei 20°C) hergestellt. Es werden folgende Werte erhalten:

1%ige Lösung: 25,7 $mN \cdot m^{-1}$

0,1%ige Lösung: 26,4 $mN \cdot m^{-1}$

Beispiel 4

Überprüfung der erfindungsgemässen Verbindungen in Haarpflegemitteln

Ein Konditioniershampoo folgender Zusammensetzung

| | |
|---|---|
| Natriumlaurylethersulfat | 3 Gew.-Teile |
| Ammoniumalkylethersulfat | 6 Gew.-Teile |
| Tagat® KL 141* | 5 Gew.-Teile |
| Betain-Siloxan (gemäss Beispiel 1) | 2 Gew.-Teile |
| Wasser | 84 Gew.-Teile |

* Handelsprodukt der Th. Goldschmidt AG

wird in seiner Wirkungsweise mit einer Shampoo-Formulierung verglichen, in der das erfindungsgemässe Betain-Siloxan durch eine kationaktive Organosiloxanverbindung gemäss DE-AS 1 493 384 mit x = 15, y = 5 und X = $Cl^{\ominus}$ ersetzt wird.

Das Präparat mit dem Betain-Siloxan ist klar, während das kationaktive Siloxan zur Eintrübung führt.

In der praktischen Anwendung beim halbseitigen Vergleichstest auf menschlichem Haar ergibt sich folgende Beurteilung:

Das erfindungsgemässe Betain-Siloxan ist in der Schaumbildung, der Cremigkeit des Schaumes, der Trockenkämmbarkeit, der antielektrostatischen Wirkung und der Fülle des Haares überlegen. Lediglich in der Nasskämmbarkeit ergibt das kationaktive Siloxan ein etwas besseres Resultat. Dies ist jedoch mit einer stärkeren Beschwerung und damit einer geringeren Fülle des Haares verbunden.

## Patentansprüche

1. Verbindungen der allgemeinen Formel

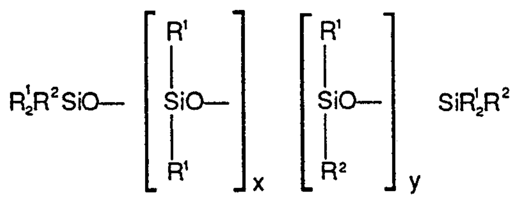

wobei

$R^1$ im Molekül gleich oder verschieden ist und einen Alkylrest mit 1 bis 18 Kohlenstoffatomen, einen Arylrest oder einen Polyoxyalkylenrest der allgemeinen Formel $-(CH_2)_3O(C_mH_{2m}O)_pQ$ bedeutet, wobei die Gruppe $-(C_mH_{2m}O)_p$ aus Oxyethylen- und Oxypropyleneinheiten aufgebaut ist, m einen durchschnittlichen Wert von 2,0 bis 2,6 p einen Wert von 1 bis 25 hat, Q ein Wasserstoff- oder ein Alkylrest mit 1 bis 4 Kohlenstoffatomen ist, jedoch mindestens 70% der Reste $R^1$ Methylreste sind,

$R^2$ gleich $R^1$ sein kann, wobei mindestens ein Rest $R^2$ die Gruppe

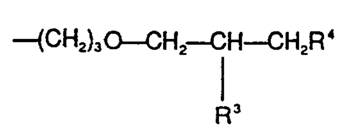

ist, in der $R^3$ und $R^4$ verschieden sind und ein Rest ein Hydroxylrest und der andere Rest die Gruppe

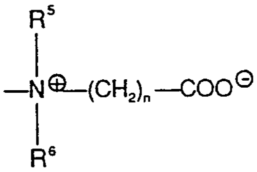

ist, in der $R^5$ und $R^6$ gleich oder verschieden sind und einen Alkylrest mit 1 bis 4 Kohlenstoffatomen oder einen Benzylrest bedeuten, und n = 1, 2 oder 3 ist,
x einen Wert von 0 bis 200 und
y einen Wert von 1 bis 50 hat.

2. Verfahren zur Herstellung der Verbindungen des Anspruchs 1, dadurch gekennzeichnet, dass man entweder

a) Verbindungen der allgemeinen Formel

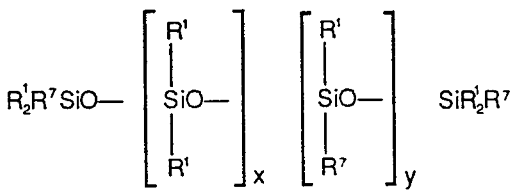

wobei $R^7$ gleich $R^1$ sein kann, mit der Massgabe, dass mindestens ein Rest $R^7$ die Gruppe

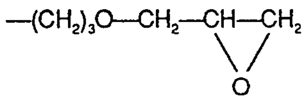

ist, mit, bezogen auf die Gruppe

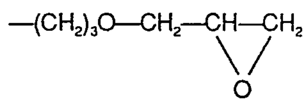

äquimolaren Mengen Verbindungen der allgemeinen Formel

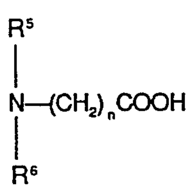

in an sich bekannter Weise umsetzt, oder

b) Verbindungen der allgemeinen Formel

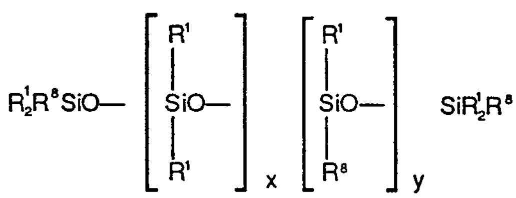

wobei $R^8$ gleich $R^1$ sein kann, mit der Massgabe, dass mindestens ein Rest $R^8$ die Gruppe

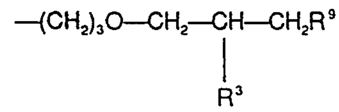

ist, in der $R^3$ und $R^9$ verschieden sind, und ein Rest ein Hydroxylrest und der andere Rest die Gruppe

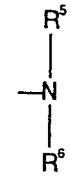

ist, mit bezogen auf die Gruppe

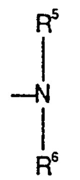
,

äquimolaren Mengen Verbindungen der allgemeinen Formel $X\text{-}(CH_2)_n\text{-}COOY$, wobei X ein Chlor- oder Bromrest und Y ein Alkalirest ist, in an sich bekannter Weise umsetzt.

3. Verfahren nach Anspruch 2, dadurch gekennzeichnet, dass man die Umsetzung a) bzw. b) in einem Lösungsmittel und/oder bei Temperaturen von 40 bis 160°C oder bis zum Siedepunkt des verwendeten Lösungsmittels durchführt.

4. Verwendung der Verbindungen des Anspruchs 1 in kosmetischen Zubereitungen, insbesondere in Zubereitungen zur Pflege der Haare.

**Claims**

1. Compounds of the general formula

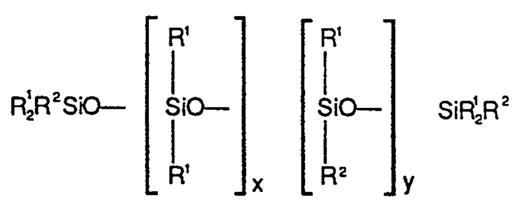

where
$R^1$ has identical or different meanings within the molecule and denotes an alkyl radical having 1 to 18 carbon atoms, an aryl radical or a polyoxyalkylene radical of the general formula $-(CH_2)_3O(C_mH_{2m}O)_pQ$ where the $-(C_mH_{2m}O)_p$ group is built up from oxyethylene and oxypropylene units, m has an average value of from 2.0 to 2.6, p has a value of from 1 to 25, and Q is a hydrogen atom or an alkyl radical having 1 to 4 carbon atoms, but at least 70% of the $R^1$ radicals are methyl radicals,
$R^2$ may be identical to $R^1$, at least one $R^2$ radical being in the

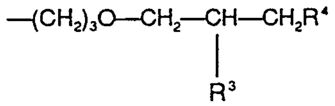

group in which $R^3$ and $R^4$ are different and one radical is a hydroxyl radical and the other radical is the

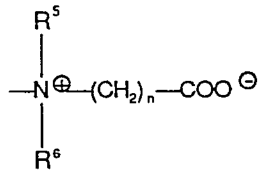

group in which $R^5$ and $R^6$ are identical or different and denote an alkyl radical having 1 to 4 carbon atoms or a benzyl radical, and n is 1, 2 or 3,
x has a value of from 0 to 200 and
y has a value of from 1 to 50.

2. Process for the preparation of the compounds of Claim 1, characterized in that either

a) compounds of the general formula

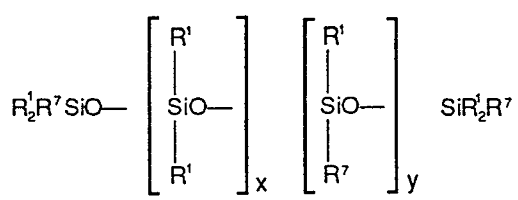

where $R^7$ may be identical to $R^1$, with the proviso that at least one $R^7$ radical is the

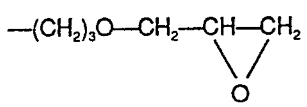

group, are reacted with equimolar amounts, based on the

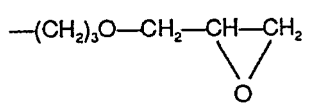

group, of compounds of the general formula

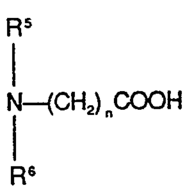

in a manner known per se, or

b) compounds of the general formula

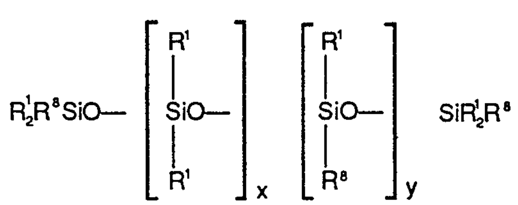

where $R^8$ may bi identical to $R^1$, with the proviso that at least on $R^8$ radical is the

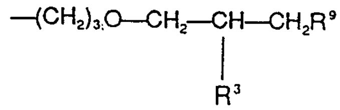

group in which $R^3$ and $R^9$ are different, and one radical is a hydroxyl radical and the other radical is the

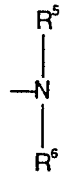

group, are reacted with equimolar amounts, based on the

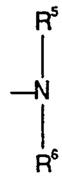

group, of compounds of the general formula $X-(CH_2)_n-COOY$ where X is a chlorine or bromine atom and Y is an alkali metal atom, in a manner known per se.

3. Process according to Claim 2, characterized in that the reaction a) or b) is carried out in a solvent and/or at temperatures of from 40 to 160°C or up to the boiling point of the solvent used.

4. Use of the compounds of Claim 1 in cosmetic preparations, in particular in preparations for hair care.

## Revendications

1. Composés de formule générale

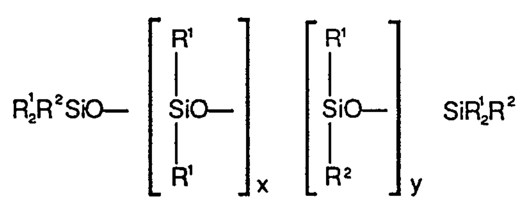

dans laquelle
les radicaux $R^1$, dans la molécule, sont identiques ou différents et représentent chacun un radical alkyle ayant de 1 à 18 atomes de carbone, un radical aryle ou un radical polyoxyalkylène de formule $-(CH_2)_3O(C_mH_{2m}O)_pQ$, où le groupe $-C_mH_{2m}O)_p$ est constitué de motifs oxyde d'éthylène et oxyde de propylène, m a pour valeur moyenne 2,0 à 2,6, p vaut de 1 à 25, Q est un hydrogène ou un radical alkyle ayant de 1 à 4 atomes de carbone, au moins 70% des radicaux $R^1$ étant cependant des radicaux méthyle,
les radicaux $R^2$ peuvent être chacun identiques à

des radicaux $R^1$, où au moins un radical $R^2$ est le groupe

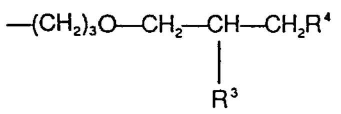

où $R^3$ et $R^4$ sont différents, et un radical est un radical hydroxy et l'autre radical est le groupe

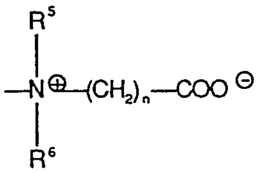

où $R^5$ et $R^6$ sont identiques ou différents et représentent chacun un radical alkyle ayant de 1 à 4 atomes de carbone ou un radical benzyle, et n = 1, 2 ou 3,
x vaut de 0 à 200 et
y vaut de 1 à 50.

2. Procédé pour la préparation de composés selon la revendication 1, caractérisé en ce qu'on fait réagir d'une manière connue en soi

a) des composés de formule générale

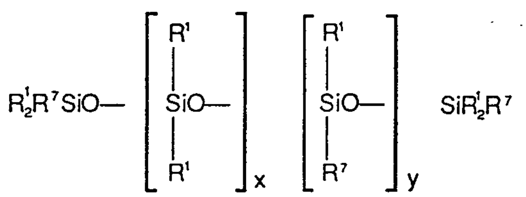

dans laquelle les radicaux $R^7$ peuvent être égaux à $R^1$, à la condition qu'au moins l'un des radicaux $R^7$ soit le groupe

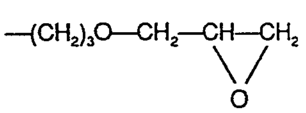

sur des quantités, équimolaires par rapport au groupe

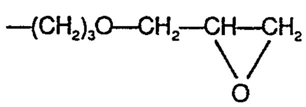

de composés de formule générale

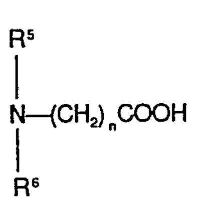

ou bien

b) des composés de formule générale

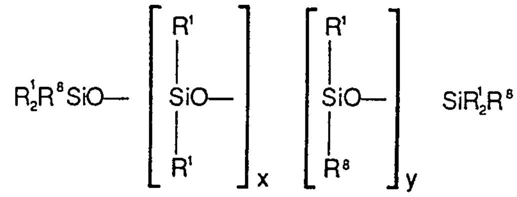

dans laquelle $R^8$ peut être égal à $R^1$, à la condition qu'au moins un radical $R^8$ soit le groupe

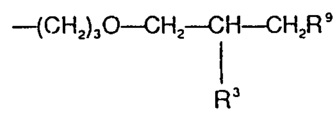

où $R^3$ et $R^9$ sont différents, et un radical est un radical hydroxyle et l'autre radical est le groupe

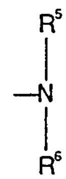

sur des quantités équimolaires, par rapport au groupe

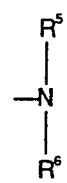

de composés de formule générale $X-(CH_2)_n-COOY$, où X est un radical chloro ou bromo et Y est un radical métal alcalin.

3. Procédé selon la revendication 2, caractérisé en ce qu'on met en œuvre la réaction a) ou b) dans un solvant et/ou à des températures de 40 à 160 °C, ou jusqu'au point d'ébullition du solvant utilisé.

4. Utilisation des composés selon la revendication 1 dans des préparations cosmétiques, en particulier dans des préparations pour les soins des cheveux.